# EUROPEAN PATENT APPLICATION

(11) **EP 2 979 654 A1**
(43) Date of publication of application: **03.02.2016**
(21) Application number: 14775972.4
(22) Date of filing: 17.03.2014
(51) Int. Cl.: A61B 18/00

(54) **ULTRASONIC TREATMENT DEVICE**

(30) Priority: 29.03.2013 JP 2013072924
(71) Applicant: Olympus Corporation, Shibuya-ku Tokyo 151-0072 (JP); The University of Tokyo, Bunkyo-ku, Tokyo 113-8654 (JP)
(72) Inventor: MURAKAMI, Miyuki, Tokyo 151-0072 (JP); YAMAGUCHI, Satoshi, Tokyo 113-8654 (JP); NAGAMUNE, Teruyuki, Tokyo 113-8654 (JP); MINAMIHATA, Kosuke, Tokyo 113-8654 (JP)
(74) Representative: Gunzelmann, Rainer
(86) International application number: PCT/JP2014/057134
(87) International publication number: WO 2014/156766

(57) **Abstract**

Treatment is provided at an ideal timing. Provided is an ultrasonic treatment apparatus (1) including: a therapeutic-ultrasound-wave radiating portion (7) that radiates therapeutic ultrasound waves for causing an ultrasonic treatment material having a tendency to accumulate in an affected area to exert a therapeutic effect; an accumulation determining portion (4b) that determines whether or not the ultrasonic treatment material has accumulated in the affected area; and a determination-result presenting portion (5) that presents a determination result obtained by the accumulation determining portion (4b).

## Description

### {Technical Field}

The present invention relates to an ultrasonic treatment apparatus.

### {Background Art}

In the related art, there are known ultrasonic treatment apparatuses using a phase-change ultrasonic contrast agent, in which nanosized droplets are administered to a living body, and ultrasound waves are radiated, thereby generating microbubbles through a phase-change of the droplets (bubble formation) (for example, see PTL 1). Like this phase-change ultrasonic contrast agent, ultrasonic contrast agents such as nanosized bubble precursors and bubble liposomes tend to pass through the walls of blood vessels, which provide nourishment to a tumor, and tend to be "passively" accumulated in the vicinity of the tumor. In the case of a nanodroplet, because it is capable of forming a microbubble when it receives ultrasound energy, the phase thereof can be changed to micrometer-sized bubbles in a site-specific manner. Furthermore, when a molecular probe, which selectively binds to molecules constituting the living body, such as an antibody or a ligand, is added to a nanosized bubble precursor or bubble liposome, the nanodroplet can be endowed with "active" tissue selectivity.

Then, therapeutic ultrasound waves are radiated in a state in which such contrast agents specifically accumulate in tissue to be treated, such as a tumor, thereby making it possible to efficiently treat the tissue to be treated, such as a tumor, due to heating promotion or a cavitation effect caused by bubble vibration. Specifically, a nanosized ultrasonic contrast agent can be used as an ultrasonic treatment material.

### {Citation List}

### {Patent Literature}

{PTL 1} Publication of Japanese Patent No. 4630127

### {Summary of Invention}

### {Technical Problem}

However, to provide treatment using this phase-change ultrasonic contrast agent, it is necessary to radiate high-intensity bubble-forming ultrasound waves, and, if irradiation is performed in a state in which the phase-change ultrasonic contrast agent has not accumulated in a site to be treated, its phase cannot be changed. Thus, it is impossible to achieve a heating enhancing effect with the therapeutic ultrasound waves; in addition, there is a disadvantage in that the phase-change ultrasonic contrast agent existing in a normal site is changed in phase, causing microbubbles, thus bringing about heating promotion or a cavitation effect in the normal tissue. Furthermore, it takes time for phase-change ultrasonic contrast agents or nanosized ultrasonic contrast agents, such as bubble liposomes, to accumulate in a treatment site, and they are eliminated through metabolism as a long time elapses after administration; therefore, it is difficult to provide treatment at an ideal timing.

The present invention has been made in view of the above-described circumstances, and an object thereof is to provide an ultrasonic treatment apparatus capable of providing treatment at an ideal timing.

### {Solution to Problem}

In order to achieve the above-described object, the present invention provides the following solutions.

According to one aspect, the present invention provides an ultrasonic treatment apparatus including: a therapeutic-ultrasound-wave radiating portion that radiates therapeutic ultrasound waves for causing an ultrasonic treatment material having a tendency to accumulate in an affected area to exert a therapeutic effect; an accumulation determining portion that determines whether or not the ultrasonic treatment material has accumulated in the affected area; and a determination-result presenting portion that presents a determination result obtained by the accumulation determining portion.

According to this aspect, after an ultrasonic treatment material having a tendency to accumulate in an affected area is administered to a patient, the accumulation determining portion is activated to determine whether or not the ultrasonic treatment material has accumulated in the affected area, and a determination result is presented by the determination-result presenting portion, thereby making it possible for a doctor who views the presented determination result to recognize an appropriate timing for radiating therapeutic ultrasound waves. Then, if it is determined that the ultrasonic treatment material has accumulated in the affected area, the therapeutic-ultrasound-wave radiating portion is activated to radiate therapeutic ultrasound waves onto the affected area, thereby making it possible to make the ultrasonic treatment material exert a therapeutic effect to efficiently treat the affected area. On the other hand, if it is determined that the ultrasonic treatment material has not accumulated therein, the therapeutic-ultrasound-wave radiating portion does not radiate therapeutic ultrasound waves, thereby making it possible to prevent the ultrasonic treatment material from being activated in a normal site and to maintain the health of the normal site.

In the above-described aspect, it is possible to include: a therapeutic-ultrasound-wave radiating portion that radiates therapeutic ultrasound waves for causing an ultrasonic treatment material having a tendency to accumulate in an affected area to exert a therapeutic effect; an accumulation determining portion that determines whether or not the ultrasonic treatment material has accumulated in the affected area; and a control portion that controls the therapeutic-ultrasound-wave radiating portion based on a determination result obtained by the accumulation determining portion.

By doing so, if it is determined in the accumulation determining portion that the ultrasonic treatment material has accumulated in the affected area, the control portion activates the therapeutic-ultrasound-wave radiating portion, and, if it is determined in the accumulation determining portion that the ultrasonic treatment material has not accumulated therein, the control portion stops the therapeutic-ultrasound-wave radiating portion, thereby making it possible to efficiently treat the affected area and to maintain the health of a normal site.

In the above-described aspect, it is possible to further include a treatment-material detecting portion that detects a signal indicating an existence of the ultrasonic treatment material, in which the accumulation determining portion may determine that the ultrasonic treatment material has accumulated in the affected area when the signal detected by the treatment-material detecting portion exceeds a predetermined threshold.

By doing so, a situation in which a predetermined level or more of the ultrasonic treatment material has accumulated in the affected area can be easily detected by the treatment-material detecting portion, and whether it is an appropriate timing for radiating the therapeutic ultrasound waves can be objectively determined by the accumulation determining portion.

In the above-described aspect, the signal indicating the existence of the ultrasonic treatment material may be a signal based on at least one of ultrasound waves that are reflected at the ultrasonic treatment material, and magnetism, light, and radiation that are produced from the ultrasonic treatment material.

By doing so, with those signals, the existence of the ultrasonic treatment material can be detected in a non-contact manner.

In the above-described aspect, it is possible to further include: an image generating portion that generates a distribution image showing a distribution of the signal indicating the existence of the ultrasonic treatment material, the signal being detected by the treatment-material detecting portion; and a display portion that displays the distribution image generated by the image generating portion.

By doing so, the distribution image of the signal that indicates the existence of the ultrasonic treatment material and that is detected by the treatment-material detecting portion, the distribution image being generated by the image generating portion, is presented to the display portion, thereby making it possible to visually recognize a treatment site.

In the above-described aspect, it is possible to further include: a storage portion that stores the signal detected by the treatment-material detecting portion before the therapeutic ultrasound waves are radiated by the therapeutic-ultrasound-wave radiating portion; and a treatment-status determining portion that determines a status of treatment by comparing a pre-treatment signal stored in the storage portion with a mid-treatment signal detected by the treatment-material detecting portion during radiation of the therapeutic ultrasound waves.

By doing so, the therapeutic ultrasound waves are radiated, thereby making it possible to crush or vibrate bubbles in the ultrasonic treatment material accumulating in the affected area and to treat the affected area using the energy thereof. In this case, on the basis of a pre-treatment signal indicating the existence of the ultrasonic treatment material before the therapeutic ultrasound waves are radiated, the pre-treatment signal being stored in the storage portion, the treatment progress can be easily determined from a reduced magnitude of a mid-treatment signal indicating the existence of the ultrasonic treatment material, the magnitude being reduced by radiation of the therapeutic ultrasound waves.

In the above-described aspect, it is possible to further include a diagnostic-ultrasound-wave radiating portion that radiates diagnostic ultrasound waves whose intensity is lower than that of the therapeutic ultrasound waves, in which the treatment-material detecting portion may detect ultrasound waves reflected at the ultrasonic treatment material.

By doing so, it is possible to radiate, from the diagnostic-ultrasound-wave radiating portion, relatively-weak ultrasound waves that are insufficient for treatment but are sufficient for detection, and to easily detect the degree of accumulation of the ultrasonic treatment material in the affected area while maintaining the health of the normal site.

In the above-described aspect, it is possible to further include: a dynamic library that stores timing data regarding pharmacokinetics of the ultrasonic treatment material from a point of time of administration thereof to a body; and a timer that measures elapsed time from a time at which the ultrasonic treatment material is administered to the body, in which the accumulation determining portion may determine that the ultrasonic treatment material has accumulated in the affected area when the elapsed time measured by the timer falls within a time range of accumulation in the affected area, in the timing data stored in the dynamic library.

By doing so, a situation in which the ultrasonic treatment material has accumulated in the affected area can be easily determined based on the elapsed time from administration, which is measured by the timer, and timing data regarding pharmacokinetics from the point of time of administration, which is stored in the dynamic library.

In the above-described aspect, it is possible to further include an insertion portion that is insertable into a body cavity of the patient, in which the therapeutic-ultrasound-wave radiating portion and the treatment-material detecting portion may be disposed at a distal end of the insertion portion.

By doing so, it is possible to insert the insertion portion into the body cavity of the patient, to detect accumulation of the ultrasonic treatment material in the affected area, at a position close to the affected area, with the distal end of the insertion portion being located in the vicinity of the affected area, and to radiate therapeutic ultrasound waves onto the affected area where the ultrasonic treatment material has accumulated, from a position close to the affected area. Thus, it is possible to improve the detection accuracy and to enhance the therapeutic effect.

### {Advantageous Effect of Invention}

According to the present invention, an advantageous effect is afforded in that treatment can be provided at an ideal timing.

### {Brief Description of Drawings}

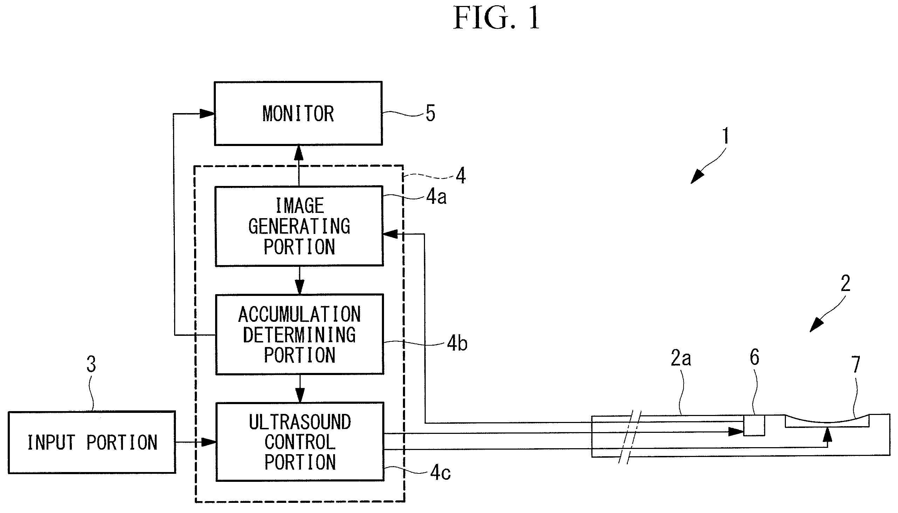

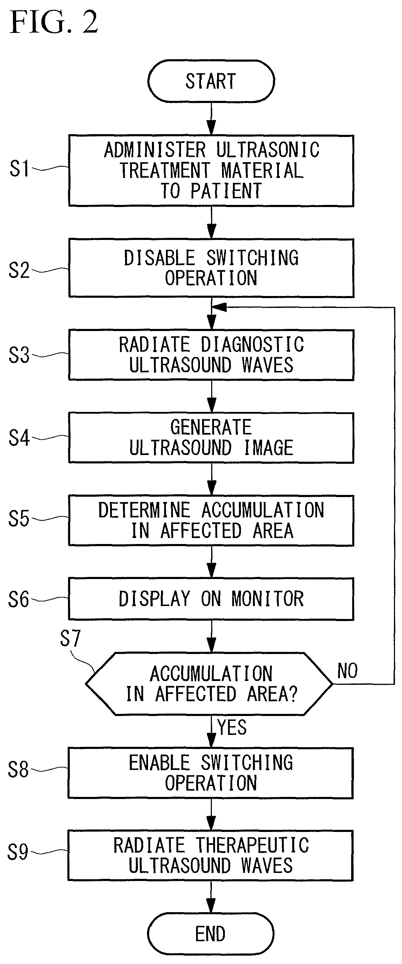

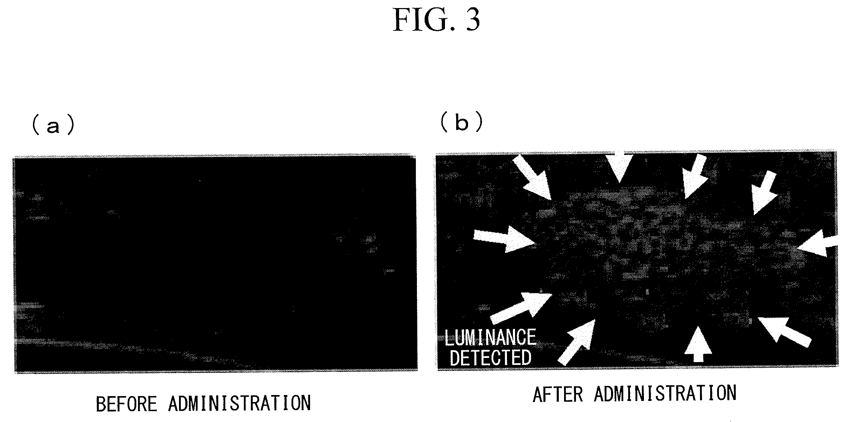

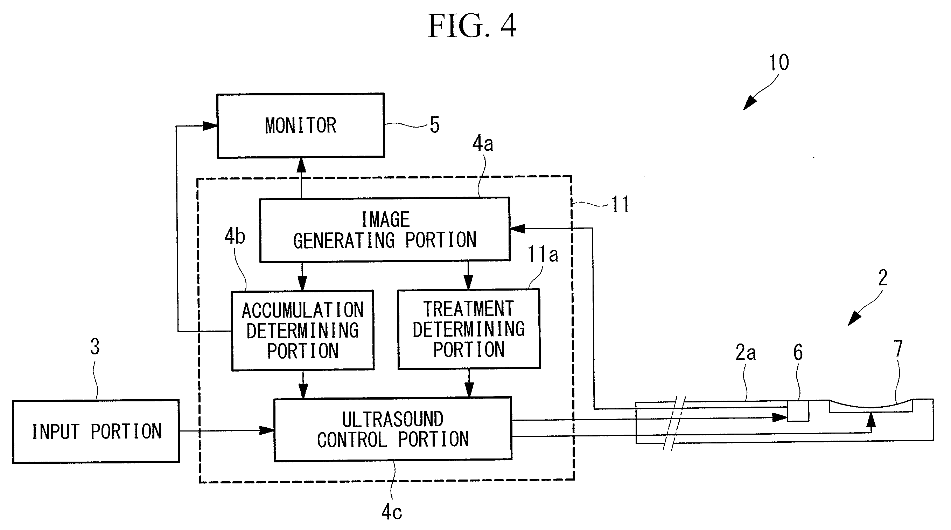

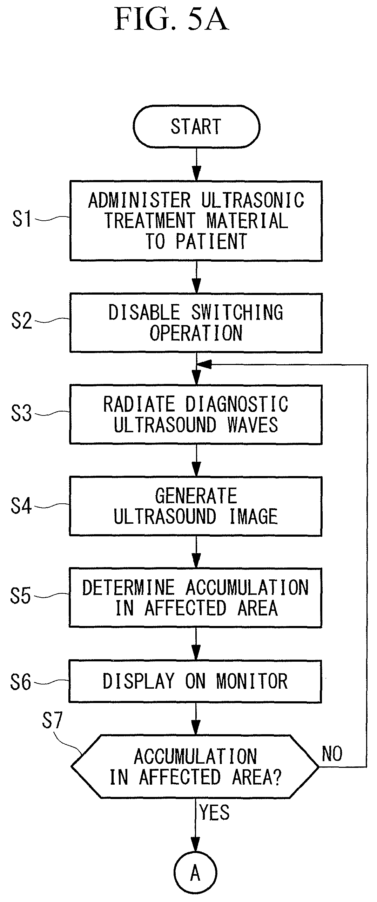

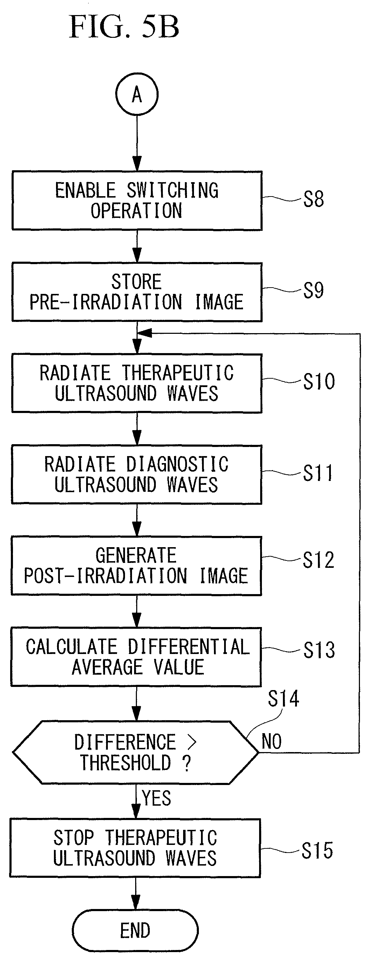

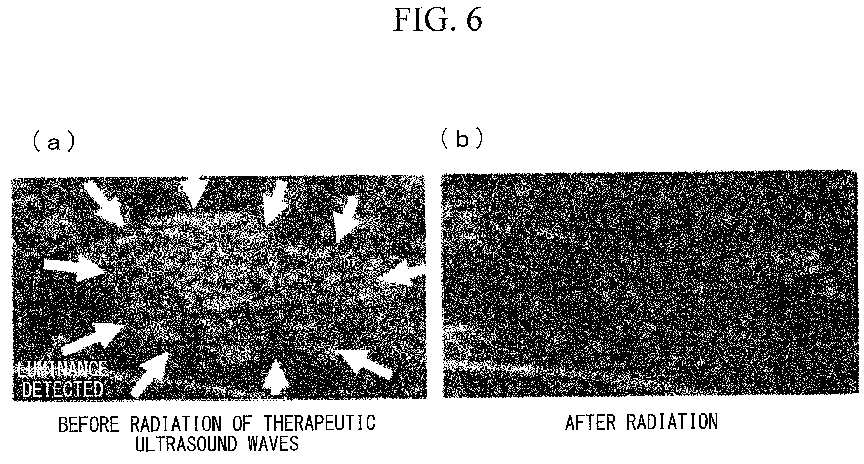

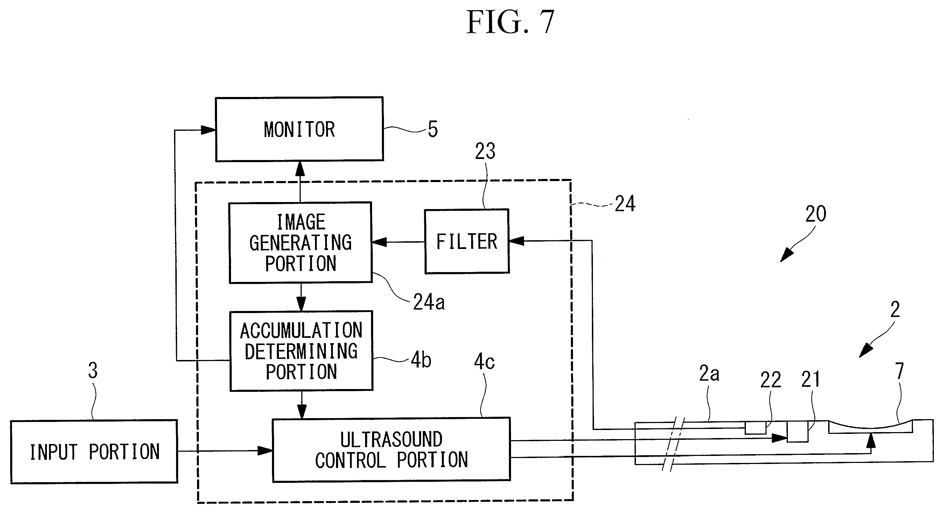

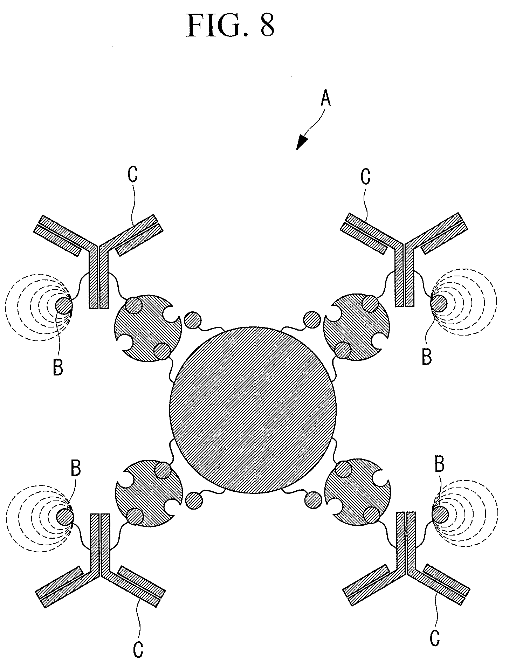

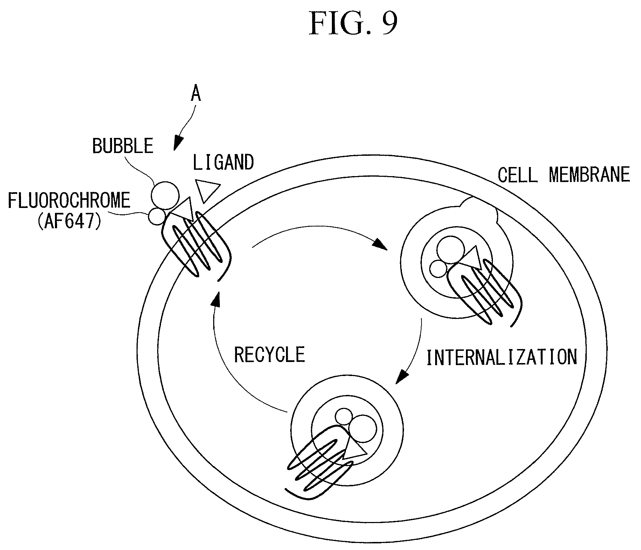

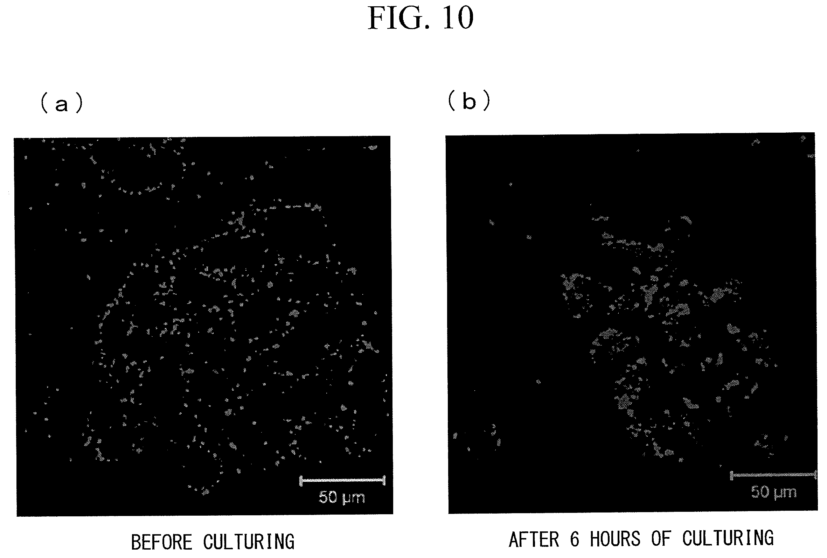

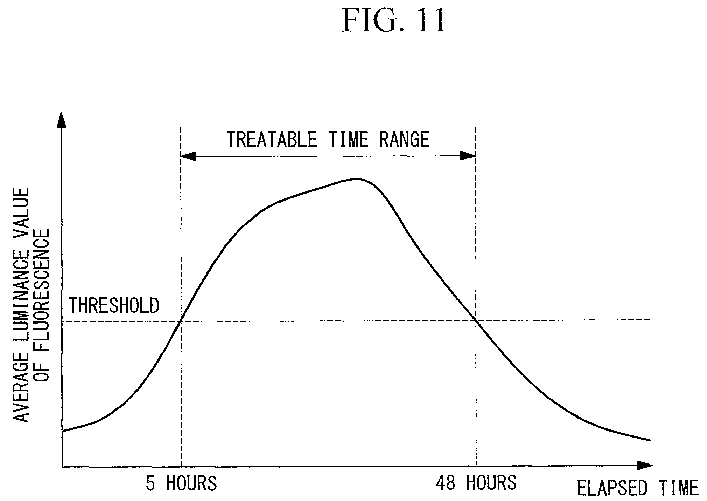

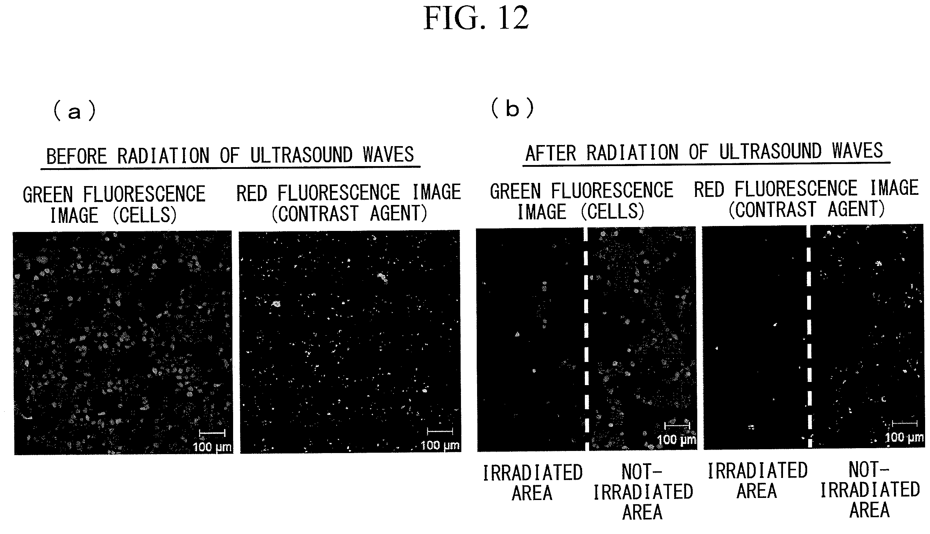

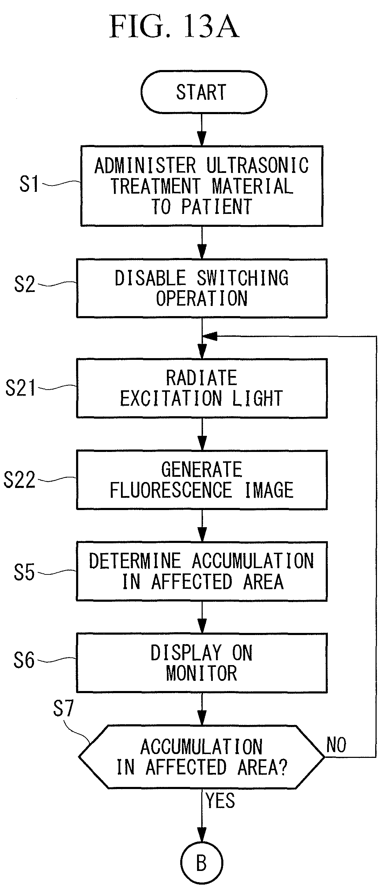

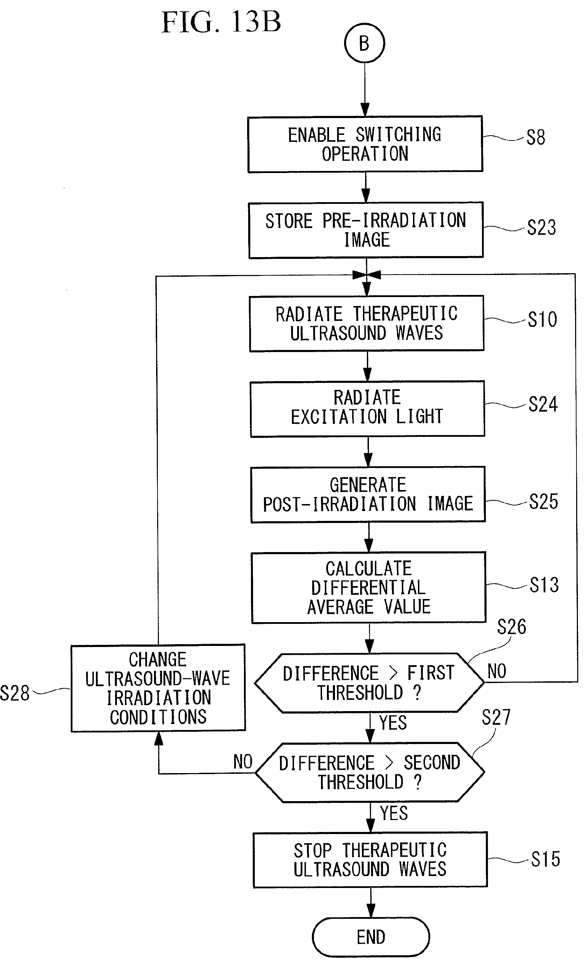

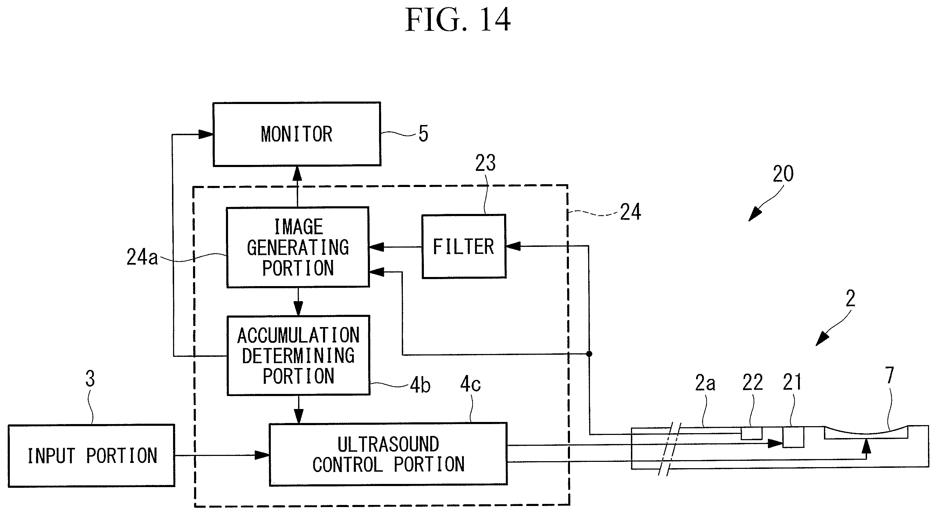

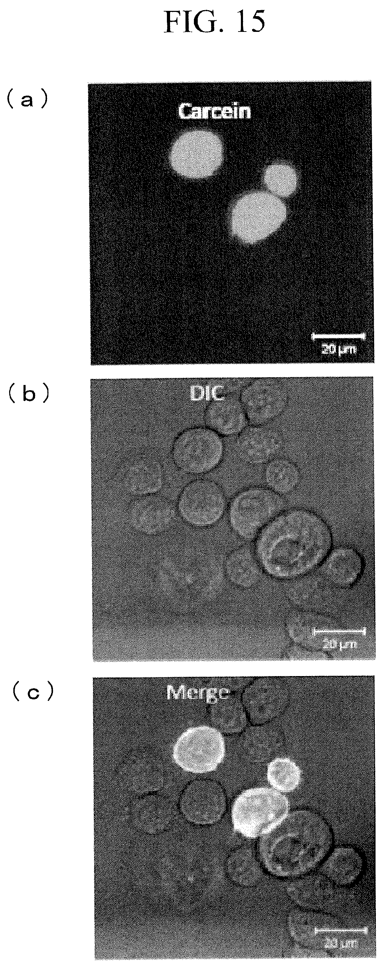

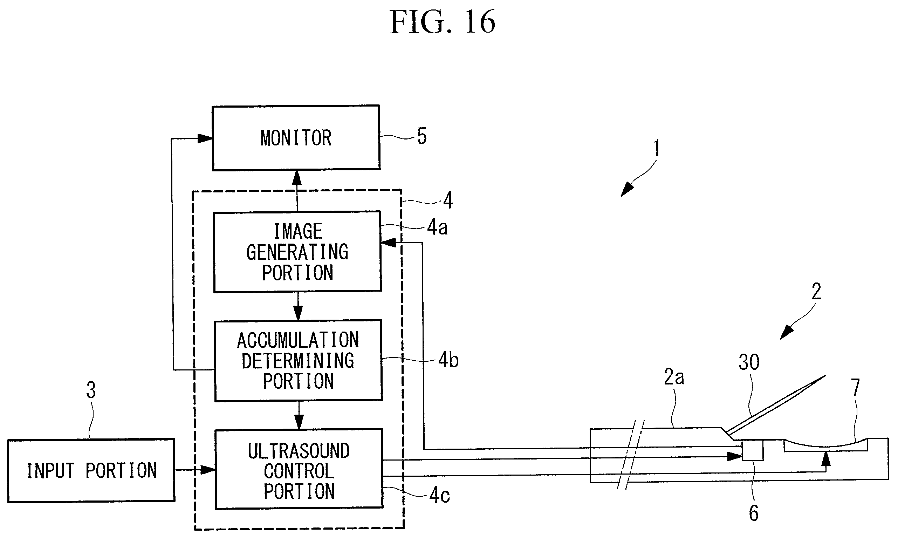

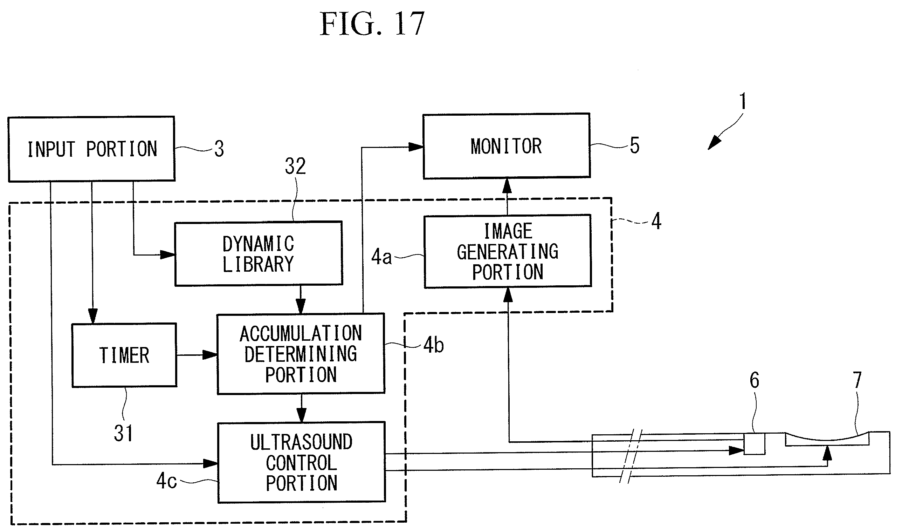

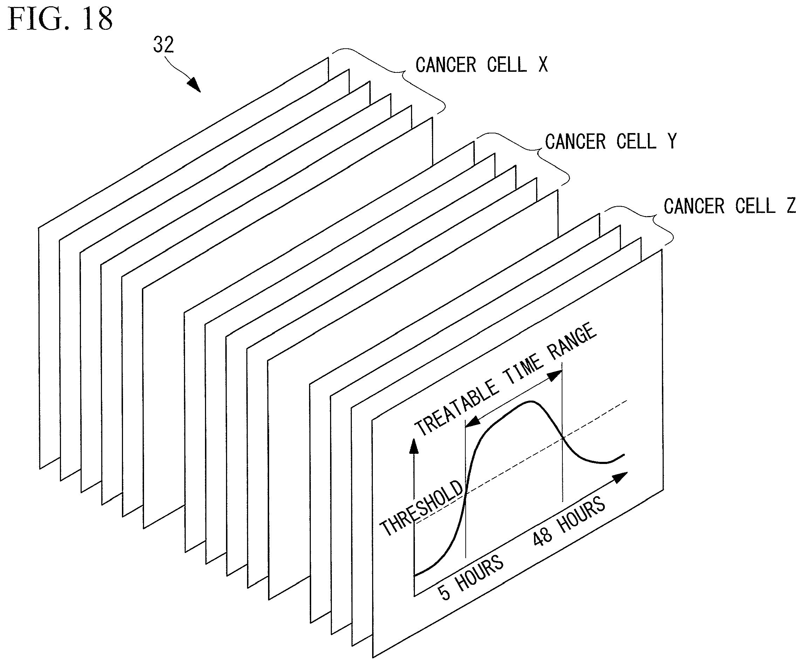

{Fig. 1} Fig. 1 is a view showing the overall configuration of an ultrasonic treatment apparatus according to a first embodiment of the present invention.
{Fig. 2} Fig. 2 is a flowchart for explaining a treatment method using the ultrasonic treatment apparatus shown in Fig. 1.
{Fig. 3} Fig. 3 shows (a) an ultrasound image before administration of an ultrasonic treatment material and (b) an ultrasound image after administration thereof, for explaining determination of accumulation of the ultrasonic treatment material in an affected area, in the ultrasonic treatment apparatus shown in Fig. 1.
{Fig. 4} Fig. 4 is a view showing the overall configuration of an ultrasonic treatment apparatus according to a second embodiment of the present invention.
{Fig. 5A} Fig. 5A is a flowchart for explaining a treatment method using the ultrasonic treatment apparatus shown in Fig. 4.
{Fig. 5B} Fig. 5B is a flowchart for explaining the treatment method using the ultrasonic treatment apparatus shown in Fig. 4.
{Fig. 6} Fig. 6 shows (a) an ultrasound image before radiation of therapeutic ultrasound waves and (b) an ultrasound image after radiation thereof, for explaining determination of a treatment status, in the ultrasonic treatment apparatus shown in Fig. 1.
{Fig. 7} Fig. 7 is a view showing the overall configuration of an ultrasonic treatment apparatus according to a third embodiment of the present invention.
{Fig. 8} Fig. 8 is a schematic diagram showing an example ultrasonic treatment material used for treatment provided by the ultrasonic treatment apparatus shown in Fig. 7.
{Fig. 9} Fig. 9 is a schematic diagram for explaining internalization of the ultrasonic treatment material shown in Fig. 8.
{Fig. 10} Fig. 10 shows (a) a fluorescence image before culturing and (b) a fluorescence image after culturing, for explaining the internalization shown in Fig. 9.
{Fig. 11} Fig. 11 is a graph showing an example timing data regarding pharmacokinetics of the ultrasonic treatment material shown in Fig. 8.
{Fig. 12} Fig. 12 shows (a) a fluorescence image before radiation of therapeutic ultrasound waves and (b) a fluorescence image after radiation thereof, for explaining determination of the status of treatment provided by the ultrasonic treatment apparatus shown in Fig. 7.
{Fig. 13A} Fig. 13A is a flowchart for explaining a treatment method using the ultrasonic treatment apparatus shown in Fig. 7.
{Fig. 13B} Fig. 13B is a flowchart for explaining the treatment method using the ultrasonic treatment apparatus shown in Fig. 7.
{Fig. 14} Fig. 14 is a view showing the overall configuration of a modification of the ultrasonic treatment apparatus shown in Fig. 7.
{Fig. 15} Fig. 15 shows (a) a fluorescence image of a specific wavelength, (b) a differential interference image, and (c) a superposed image obtained by superposing the fluorescence image and the differential interference image, which are acquired by the ultrasonic treatment apparatus shown in Fig. 14.
{Fig. 16} Fig. 16 is a view showing the overall configuration of a modification of the ultrasonic treatment apparatus shown in Fig. 1.
{Fig. 17} Fig. 17 is a view showing the overall configuration of another modification of the ultrasonic treatment apparatus shown in Fig. 1.
{Fig. 18} Fig. 18 is a view showing an example dynamic library provided in the ultrasonic treatment apparatus shown in Fig. 17.

### {Description of Embodiments}

An ultrasonic treatment apparatus 1 according to a first embodiment of the present invention will be described below with reference to the drawings.

As shown in Fig. 1, the ultrasonic treatment apparatus 1 of this embodiment includes a treatment-apparatus main body 2, an input portion 3 through which an operator, such as a doctor, performs input, a control portion 4 that controls the treatment-apparatus main body 2, and a monitor 5 that displays information acquired by the treatment-apparatus main body 2.

The treatment-apparatus main body 2 includes, at the distal end of an elongated insertion portion 2a to be inserted into a body cavity, a first ultrasonic vibrator 6 that emits diagnostic ultrasound waves radially outward and that receives an echo and a second ultrasonic vibrator 7 that emits therapeutic ultrasound waves radially outward. The second ultrasonic vibrator 7 emits focused ultrasound waves. Ultrasound waves emitted from the first ultrasonic vibrator 6 are set to be sufficiently low in intensity compared with ultrasound waves emitted from the second ultrasonic vibrator 7.

The input portion 3 is a switch for activating the first ultrasonic vibrator 6 and the second ultrasonic vibrator 7.

The control portion 4 includes an image generating portion 4a that generates an image of ultrasound waves received by the first ultrasonic vibrator 6, an accumulation determining portion 4b that determines whether or not an ultrasonic treatment material has accumulated based on the luminance value of the ultrasound image generated by the image generating portion 4a, and an ultrasound control portion 4c that enables or disables a switching operation for activating the second ultrasonic vibrator 7 based on a determination result output from the accumulation determining portion 4b.

The accumulation determining portion 4b determines that the ultrasonic treatment material has accumulated when the average value of luminance values in a predetermined region of the ultrasound image generated by the image generating portion 4a exceeds a predetermined threshold.

The ultrasound control portion 4c enables the switching operation performed through the input portion 3 when the accumulation determining portion 4b outputs a determination result indicating that the ultrasonic treatment material has accumulated and disables the switching operation performed through the input portion 3 when the accumulation determining portion 4b outputs a determination result indicating that the ultrasonic treatment material has not accumulated. A switching operation performed through the input portion 3 for activating the first ultrasonic vibrator 6 is always disabled.

The monitor 5 displays an ultrasound image generated by the image generating portion 4a and also displays a determination result output from the accumulation determining portion 4b.

The operation of the thus-configured ultrasonic treatment apparatus 1 of this embodiment will be described below.

To provide treatment for an affected area by using the ultrasonic treatment apparatus 1 of this embodiment, as shown in Fig. 2, an ultrasonic treatment material is first administered to a patient (Step S1). At this point, input through the input portion 3 for activating the second ultrasonic vibrator 7 is set to be disabled (Step S2).

The ultrasonic treatment material is a liquid containing nanosized air bubbles (several tens of nm to several hundreds of nm), for example, and is administered to the body of the patient by intravenous injection, for example. After being administered, the ultrasonic treatment material accumulates in an affected area, such as a tumor, where angiogenesis often exists, with time by the EPR effect.

Next, the operator, such as a doctor, inserts the insertion portion 2a into the body cavity of the patient and positions the first ultrasonic vibrator 6 and the second ultrasonic vibrator 7, which are disposed at the distal end of the insertion portion 2a, so as to face the affected area.

Then, the operator operates the input portion 3 to activate the first ultrasonic vibrator 6 (Step S3). Accordingly, diagnostic ultrasound waves are emitted by the first ultrasonic vibrator 6, and an ultrasound image is generated in the image generating portion 4a from an echo received when the diagnostic ultrasound waves are reflected at the affected area and return (Step S4).

Furthermore, the ultrasound image generated in the image generating portion 4a is sent to the accumulation determining portion 4b. Then, the accumulation determining portion 4b determines whether the luminance average value in a predetermined region exceeds the predetermined threshold (Step S5), and the determination result and the ultrasound image are displayed on the monitor 5 (Step S6). If the determination result indicates no accumulation, the process steps from Step S3 are repeated (Step S7).

Fig. 3 shows (a) an ultrasound image showing a state in which the ultrasonic treatment material has not accumulated and (b) an ultrasound image showing a state in which the ultrasonic treatment material has accumulated. As indicated by arrows in Fig. 3(b), portions where the ultrasonic treatment material has accumulated have high luminance in the ultrasound image.

After recognizing the ultrasound image and the determination result (for example, the words "luminance detected") displayed on the monitor 5, the operator operates the input portion 3.

If the determination result output from the accumulation determining portion 4b indicates that the ultrasonic treatment material has accumulated in the affected area, the ultrasound control portion 4c in the control portion 4 enables the switching operation for activating the second ultrasonic vibrator 7 (Steps S7 and S8); therefore, when the operator operates the input portion 3, the second ultrasonic vibrator 7 can be activated.

Accordingly, therapeutic ultrasound waves are output from the second ultrasonic vibrator 7 (Step S10), and nanosized air bubbles in the ultrasonic treatment material are vibrated or crushed, thus making it possible to provide treatment for the affected area, where the ultrasonic treatment material has accumulated, due to heating promotion or a cavitation effect.

In this case, according to the ultrasonic treatment apparatus 1 of this embodiment, prior to activation of the second ultrasonic vibrator 7, an ultrasound image is acquired from diagnostic ultrasound waves emitted from the first ultrasonic vibrator 6, it is determined whether or not the ultrasonic treatment material has accumulated in the affected area, and the determination result is displayed on the monitor 5.

Therefore, after viewing the monitor 5, the operator can perform an operation so as to radiate therapeutic ultrasound waves in a state in which the ultrasonic treatment material has sufficiently accumulated in the affected area, to efficiently treat the affected area. Furthermore, it is possible to prevent wastefully radiating the therapeutic ultrasound waves in a state in which the ultrasonic treatment material has not accumulated. Furthermore, it is possible to prevent a disadvantage in that the high-intensity therapeutic ultrasound waves are radiated in a state in which the ultrasonic treatment material is distributed at a normal site, thus maintaining the health of the normal site.

Furthermore, according to this embodiment, the ultrasound control portion 4c disables the operation of the second ultrasonic vibrator 7 based on the determination result indicating that the ultrasonic treatment material has not accumulated in the affected area, which is output from the accumulation determining portion 4b; therefore, even if the operator tries to emit therapeutic ultrasound waves by mistake, the second ultrasonic vibrator 7 is not activated, thus making it possible to prevent the occurrence of the above-described disadvantage.

In this embodiment, although the ultrasound control portion 4c enables or disables the switching operation performed through the input portion 3, instead of this, the ultrasound control portion 4c may activate the second ultrasonic vibrator 7 depending on the determination result output from the accumulation determining portion 4b. Specifically, when the operator who views the ultrasound image and the determination result displayed on the monitor 5 gives an instruction to activate the second ultrasonic vibrator 7 through the input portion 3, the ultrasound control portion 4c may wait for a determination result indicating that the ultrasonic treatment material has accumulated in the affected area and then activate the second ultrasonic vibrator 7.

Furthermore, without using a command signal for emitting therapeutic ultrasound waves sent from the input portion 3, the ultrasound control portion 4c may automatically activate the second ultrasonic vibrator 7 depending on the determination result output from the accumulation determining portion 4b. In this case, the operator may perform an operation for stopping radiation of the therapeutic ultrasound waves, through the input portion 3. Then, the operator or the ultrasound control portion 4c may change the therapeutic-ultrasound-wave irradiation conditions based on the luminance value in an ultrasound image acquired after one cycle of ultrasound-wave irradiation.

Furthermore, in this embodiment, the first ultrasonic vibrator 6 and the second ultrasonic vibrator 7 are separately provided, and the diagnostic ultrasound waves and the therapeutic ultrasound waves are separately output; however, instead of this, a single ultrasonic vibrator may be switched to output diagnostic ultrasound waves or therapeutic ultrasound waves. Accordingly, the space where the ultrasonic vibrators are installed at the distal end of the insertion portion 2a is reduced, thus making it possible to achieve a reduction in the size of the apparatus.

Next, an ultrasonic treatment apparatus 10 according to a second embodiment of the present invention will be described below with reference to the drawings.

In the description of the ultrasonic treatment apparatus 10 of this embodiment, identical reference signs are assigned to portions having the same configurations as those in the above-described ultrasonic treatment apparatus 1 of the first embodiment, and a description thereof will be omitted.

As shown in Fig. 4, the ultrasonic treatment apparatus 10 of this embodiment differs from the ultrasonic treatment apparatus 1 of the first embodiment in that a control portion 11 includes a treatment determining portion 11a that determines the progress of treatment based on an ultrasound image generated by the image generating portion 4a.

The treatment determining portion 11a stores an ultrasound image acquired before therapeutic ultrasound waves are radiated (pre-irradiation image), calculates differences between an ultrasound image generated while therapeutic ultrasound waves are being radiated (post-irradiation image) and the pre-irradiation image, and determines that the treatment has been completed when the average value of the calculated differences exceeds a predetermined threshold.

The operation of the thus-configured ultrasonic treatment apparatus 10 of this embodiment will be described below.

To provide treatment for the affected area by using the ultrasonic treatment apparatus 10 of this embodiment, as shown in Figs. 5A and 5B, the process steps up to determination of whether or not the ultrasonic treatment material has accumulated in the affected area is determined by the accumulation determining portion 4b, and the switching operation is switched between enabled and disabled (Step S8), are the same as those in the first embodiment. If the accumulation determining portion 4b determines that the ultrasonic treatment material has accumulated in the affected area, and the switching operation is switched to be enabled (Step S8), the control portion 11 stores the latest ultrasound image generated in the image generating portion 4a as a pre-irradiation image (Step S9).

After that, the ultrasound control portion 4c activates the second ultrasonic vibrator 7, thus radiating therapeutic ultrasound waves onto the affected area (Step S10). Then, after radiation of the therapeutic ultrasound waves, the first ultrasonic vibrator 6 is activated again, thus radiating diagnostic ultrasound waves onto the affected area (Step S11) and generating an ultrasound image (post-irradiation image) (Step S12).

When the post-irradiation image is generated, the average value of differences between this post-irradiation image and the stored pre-irradiation image is calculated (Step S13), and it is determined whether the average value of differences exceeds the predetermined threshold (Step S14).

Fig. 6 shows (a) an example pre-irradiation image and (b) an example post-irradiation image. Portions where the ultrasonic treatment material has accumulated have high luminance in the ultrasound image, as indicated by arrows in Fig. 6(a), whereas, after radiation of the therapeutic ultrasound waves, they have low luminance in the ultrasound image, as shown in Fig. 6(b), because the state of the ultrasonic treatment material has been changed due to spreading or denaturing thereof, thus increasing the differences between the pre-irradiation image and the post-irradiation image.

If the average value of the differences exceeds the threshold, the ultrasound control portion 4c determines that the treatment has been completed and stops the activation of the second ultrasonic vibrator 7 (Step S15). If the average value of the differences does not exceed the predetermined threshold, the process steps from Step S10 are repeated.

As described above, according to the ultrasonic treatment apparatus 10 of this embodiment, because radiation of therapeutic ultrasound waves is adjusted depending on the treatment progress, it is possible to prevent a disadvantage in that the therapeutic ultrasound waves continue to be radiated onto the affected area even after completion of the treatment.

Next, an ultrasonic treatment apparatus 20 according to a third embodiment of the present invention will be described below with reference to the drawings.

As shown in Fig. 7, the ultrasonic treatment apparatus 20 of this embodiment includes, instead of the first ultrasonic vibrator 6, which emits and detects diagnostic ultrasound waves, a light source 21 that produces excitation light, a light detector 22 that detects fluorescence, and a filter 23 that transmits only light of a specific fluorescence wavelength, in the light detected by the light detector 22. Furthermore, an image generating portion for generating a fluorescence image, instead of generating an ultrasound image, is provided as an image generating portion 24a of a control portion 24.

Specifically, instead of radiating diagnostic ultrasound waves and detecting an echo, excitation light is radiated from the light source 21, and fluorescence produced in the affected area is detected by the light detector 22. Accordingly, a fluorescence image of the vicinity of the affected area can be generated.

As a prerequisite for treating the affected area by using the ultrasonic treatment apparatus 20 of this embodiment, a treatment material labeled with fluorochrome B is used as an ultrasonic treatment material A to be administered to the patient. Furthermore, as shown in Fig. 8, the treatment material may be labeled with antibodies C that are specifically bound to the affected area, which is the treatment target.

The operation of the thus-configured ultrasonic treatment apparatus 20 of this embodiment will be described below.

To provide treatment for the affected area by using the ultrasonic treatment apparatus 20 of this embodiment, the ultrasonic treatment material A is first administered to the patient.

As shown in Fig. 9, when reaching the affected area, the ultrasonic treatment material A labeled with the antibodies C and the fluorochrome B is ingested into cells from cell surfaces of the affected area through internalization. Internalization means the phenomenon of ligand-bound receptors being ingested into cells from cell surfaces.

After the receptors are transferred into the cells, ligands are removed from the receptors, and thus the receptors appear on cell membrane surfaces again. Because the receptors are recycled, they do not remain in the cells. On the other hand, the fluorochrome-B-containing ultrasonic treatment material A, once ingested into the cells, remains in endosomes for a long time. If there are a plurality of ligand-labeled ultrasonic treatment materials A, they are successively ingested into the cells and are aggregated in the endosomes with time.

Fig. 10 shows (a) a fluorescence image acquired before culturing on a petri dish and (b) a fluorescence image acquired after 6 hours of culturing thereon. It is understood from Fig. 10(b) that the fluorochrome is aggregated through internalization.

Then, as time further passes, the ultrasonic treatment material A is eventually eliminated from the cells and the body through the metabolic action of the living body.

Specifically, the aggregated level and the eliminated level of the ultrasonic treatment material A labeled with the antibodies C and the fluorochrome B can be quantified based on the luminance pattern, the size, the integrated luminance value, etc., in a predetermined ROI.

Fig. 11 shows an example time course of internalization. According to this, the period of time in which the fluorescence intensity of the fluorochrome B exceeds a predetermined threshold indicates the period of time in which the fluorochrome-B-containing ultrasonic treatment material A ingested into the cells remains in the endosomes. By radiating the therapeutic ultrasound waves during this period of time, the ultrasonic treatment material A can be crushed or vibrated in the cells.

In general, cells have the property that they are resistant to external stimuli but are sensitive to internal stimuli. Specifically, there is the advantage that, when the treatment is started according to the time course of internalization, it is possible to bring about cell death by giving internal stimuli to cells having a lesion, such as a tumor, and to achieve a good therapeutic effect. If the ultrasonic treatment material A is a phase-change ultrasonic contrast agent, ultrasound waves for phase change are radiated as the therapeutic ultrasound waves during the period of time in which the phase-change ultrasonic contrast agent remains in the endosomes, thereby making it possible to bring about cell death due to a "phase change" in the cells.

Furthermore, as shown in Fig. 12, by comparing (a) fluorescence images acquired before radiation of therapeutic ultrasound waves with (b) fluorescence images acquired after radiation thereof, it is understood that the luminance of fluorescence disappears from the acquired fluorescence images because the fluorochrome therein spreads in an early stage due to cytolysis. Accordingly, the status of the treatment can be checked.

Therefore, according to the ultrasonic treatment apparatus 20 of this embodiment, as shown in Fig. 13A, excitation light is radiated (Step S21), and a fluorescence image is generated by detecting fluorescence produced from the fluorochrome modified by the ultrasonic treatment material A (Step S22). Based on the average value of fluorescence intensities in a predetermined ROI in the thus-acquired fluorescence image, the accumulation of the ultrasonic treatment material A in the affected area is determined (Steps S5 to S7).

After that, if it is determined that the ultrasonic treatment material A has accumulated in the affected area, as shown in Fig. 13B, the operation for activating the second ultrasonic vibrator 7 is enabled (Step S8), the latest fluorescence image (pre-irradiation image) is stored (Step S23), and the therapeutic ultrasound waves are radiated (Step S10). If it is determined that the ultrasonic treatment material A has not accumulated, as shown in Fig. 13A, the process steps from Step S21 are repeated.

When the therapeutic ultrasound waves are radiated onto the affected area, excitation light is radiated from the light source 21 during the radiation of the therapeutic ultrasound waves or during gaps between irradiation cycles (Step S24), a fluorescence image (post-irradiation image) is generated from fluorescence detected by the light detector 22 (Step S25), and the difference between fluorescence-intensity average values before and after radiation of the therapeutic ultrasound waves in a particular ROI is calculated (Step S13).

If the difference therebetween is equal to or lower than a first threshold, the therapeutic effect has not been achieved, and thus the process steps from Step S10 are repeated (Step S26). If the difference therebetween exceeds the first threshold but is equal to or lower than a second threshold, because the therapeutic effect is not enough, the irradiation conditions are changed, for example, by increasing the intensity of the ultrasound waves (Step S28), and then the process steps from Step S10 are repeated (Step S27).

If the difference therebetween exceeds the second threshold, because the fluorescence intensity is sufficiently reduced, it is possible to determine that the fluorochrome has spread due to cytolysis and to stop the treatment (Step S15). The end of the treatment may be determined by the operator or may be automatically determined by the control portion.

In the above-described embodiment, although the fluorescence-intensity average value is presented on the monitor 5 or is determined by the control portion 24, instead of this, as shown in Fig. 14, the image generating portion 4a may generate, in addition to fluorescence images, images from light of all wavelength bands detected by the light detector 22, without causing the light to pass through the filter 23. Accordingly, as in fluorochrome-accumulated images shown in Fig. 15, (a) a fluorescence image of a specific wavelength and (c) a superposed image obtained by superposing the fluorescence image and a differential interference image can be displayed on the monitor 5. It is also possible to display only a differential interference image shown in Fig. 15(b) by subtracting the fluorescence image shown in Fig. 15(a) from the superposed image shown in Fig. 15(c).

Furthermore, in this embodiment, although the ultrasonic treatment material A is labeled with the fluorochrome B, instead of this, it may be labeled with another label that can be detected in a non-contact manner, for example, a magnetic material or a radiation source.

Furthermore, in the above-described embodiment, although a description has been given of a case in which the ultrasonic treatment material A is administered in advance to the patient by intravenous injection etc., instead of this, as shown in Fig. 16, a channel (not shown) through which a puncture needle 30 for injecting the ultrasonic treatment material can protrude may be provided at a distal end portion of the insertion portion 2a. With this structure, the puncture needle 30 is made to protrude through the channel, thereby making it possible to directly supply the ultrasonic treatment material to the vicinity of the affected area.

As a result, the antibodies become unnecessary, thus making it possible to reduce the cost of the ultrasonic treatment material. Furthermore, compared with administration by intravenous injection, it is possible to shorten the time from administration to internalization, thus achieving a reduction in treatment time.

Furthermore, in the above-described respective embodiments, although an appropriate timing for radiating therapeutic ultrasound waves is measured through detection of diagnostic ultrasound waves or detection of fluorescence, instead of this, as shown in Fig. 17, a timer 31 used to measure elapsed time from the time of administration of the ultrasonic treatment material to the patient and a dynamic library 32 may be further included, and the accumulation determining portion 4b may determine accumulation of the ultrasonic treatment material based on the elapsed time measured by the timer 31 and based on the dynamic library 32.

Here, the dynamic library 32 is timing data showing dynamics of the ultrasonic treatment material in the body from administration to elimination and is stored for each of various types of markers (target molecules) or for each of various types of ultrasonic treatment materials, with respect to each of various types of cancer cells, as shown in Fig. 18.

The timing data stored in the dynamic library 32 is selected depending on the type of cancer cell or the type of ultrasonic treatment material, input through the input portion 3. Then, when the elapsed time from administration of the ultrasonic treatment material, which is measured by the timer 31, falls within an accumulation time range in the selected timing data, the operation of the second ultrasonic vibrator 7 is enabled, and, when the elapsed time does not fall within the accumulation time range, the operation of the second ultrasonic vibrator 7 is disabled.

By doing so, it is possible to present the timing of the start of treatment based on the timing data, which is acquired in advance, without detecting diagnostic ultrasound waves or fluorescence, and to provide treatment for the affected area with high efficiency. In particular, because a sensor or the like for determining accumulation of the ultrasonic treatment material becomes unnecessary, the cost of the ultrasonic treatment apparatus 1 can be reduced.

In the present invention, the ultrasonic treatment apparatuses 1, 10, and 20 have been described; however, the present invention is not limited thereto, and the following aspects can be derived.

### (Feature 1)

An ultrasound treatment method comprising:
an administration step of administering an ultrasonic treatment material to a patient;
a determination step of determining accumulation of the ultrasonic treatment material in an affected area; and
an irradiation step of radiating therapeutic ultrasound waves onto the affected area based on a determination result obtained in the determination step.

### (Feature 2)

The ultrasound treatment method according to feature 1, further comprising a detection step of detecting a signal caused by the ultrasonic treatment material,
wherein, in the determination step, accumulation of the ultrasonic treatment material in the affected area is determined based on the signal detected in the detection step.

### (Feature 3)

The ultrasound treatment method according to feature 1 or 2, further comprising a presentation step of presenting a determination result obtained in the determination step to an operator.

### (Feature 4)

The ultrasound treatment method according to feature 2,
wherein, in the detection step, detection is performed before and after radiation of the therapeutic ultrasound waves; and
a treatment-status determination step of determining the progress of treatment based on the signal detected in the detection step is further included.

### (Feature 5)

The ultrasound treatment method according to feature 4, further comprising a treatment-status presentation step of presenting a determination result obtained in the treatment-status determination step to an operator.

### {Reference Signs List}

- A: ultrasonic treatment material
- 1, 10, 20: ultrasonic treatment apparatus
- 2a: insertion portion
- 4: control portion
- 4a: image generating portion
- 4b: accumulation determining portion
- 5: monitor (determination-result presenting portion, display portion)
- 6: first ultrasonic vibrator (treatment-material detecting portion, diagnostic-ultrasound-wave radiating portion)
- 7: second ultrasonic vibrator (therapeutic-ultrasound-wave radiating portion)
- 11a: treatment determining portion (storage portion, treatment-status determining portion)
- 22: light detector (treatment-material detecting portion)
- 31: timer
- 32: dynamic library

## Claims

1. An ultrasonic treatment apparatus comprising:
a therapeutic-ultrasound-wave radiating portion that radiates therapeutic ultrasound waves for causing an ultrasonic treatment material having a tendency to accumulate in an affected area to exert a therapeutic effect;
an accumulation determining portion that determines whether or not the ultrasonic treatment material has accumulated in the affected area; and
a determination-result presenting portion that presents a determination result obtained by the accumulation determining portion.

2. An ultrasonic treatment apparatus comprising:
a therapeutic-ultrasound-wave radiating portion that radiates therapeutic ultrasound waves for causing an ultrasonic treatment material having a tendency to accumulate in an affected area to exert a therapeutic effect;
an accumulation determining portion that determines whether or not the ultrasonic treatment material has accumulated in the affected area; and
a control portion that controls the therapeutic-ultrasound-wave radiating portion based on a determination result obtained by the accumulation determining portion.

3. The ultrasonic treatment apparatus according to claim 1 or 2, further comprising a treatment-material detecting portion that detects a signal indicating an existence of the ultrasonic treatment material,
wherein the accumulation determining portion determines that the ultrasonic treatment material has accumulated in the affected area when the signal detected by the treatment-material detecting portion exceeds a predetermined threshold.

4. The ultrasonic treatment apparatus according to claim 3, wherein the signal indicating the existence of the ultrasonic treatment material is a signal based on at least one of ultrasound waves that are reflected at the ultrasonic treatment material, and magnetism, light, and radiation that are produced from the ultrasonic treatment material.

5. The ultrasonic treatment apparatus according to claim 3 or 4, further comprising:
an image generating portion that generates a distribution image showing a distribution of the signal indicating the existence of the ultrasonic treatment material, the signal being detected by the treatment-material detecting portion; and
a display portion that displays the distribution image generated by the image generating portion.

6. The ultrasonic treatment apparatus according to any one of claims 3 to 5, further comprising:
a storage portion that stores the signal detected by the treatment-material detecting portion before the therapeutic ultrasound waves are radiated by the therapeutic-ultrasound-wave radiating portion; and
a treatment-status determining portion that determines a status of treatment by comparing a pre-treatment signal stored in the storage portion with a mid-treatment signal detected by the treatment-material detecting portion during radiation of the therapeutic ultrasound waves.

7. The ultrasonic treatment apparatus according to any one of claims 3 to 6, further comprising a diagnostic-ultrasound-wave radiating portion that radiates diagnostic ultrasound waves whose intensity is lower than that of the therapeutic ultrasound waves,
wherein the treatment-material detecting portion detects ultrasound waves reflected at the ultrasonic treatment material.

8. The ultrasonic treatment apparatus according to any one of claims 1 to 7, further comprising:
a dynamic library that stores timing data regarding pharmacokinetics of the ultrasonic treatment material from a point of time of administration thereof to a body; and
a timer that measures elapsed time from a time at which the ultrasonic treatment material is administered to the body,
wherein the accumulation determining portion determines that the ultrasonic treatment material has accumulated in the affected area when the elapsed time measured by the timer falls within a time range of accumulation in the affected area, in the timing data stored in the dynamic library.

9. The ultrasonic treatment apparatus according to any one of claims 1 to 8, further comprising an insertion portion that is insertable into a body cavity of a patient,
wherein the therapeutic-ultrasound-wave radiating portion and the treatment-material detecting portion are disposed at a distal end of the insertion portion.
